# EUROPEAN PATENT APPLICATION

(11) **EP 3 778 616 A1**
(43) Date of publication of application: **17.02.2021**
(21) Application number: 19796157.6
(22) Date of filing: 07.05.2019
(51) Int. Cl.: C07H 1/00, C07H 1/02, C07H 21/04

(54) **OPTICALLY ACTIVE SEGMENT FOR STEREOCONTROLLED OLIGONUCLEOTIDE SYNTHESIS, PRODUCTION METHOD FOR SAME, AND STEREOCONTROLLED OLIGONUCLEOTIDE SYNTHESIS METHOD USING SAME**

(30) Priority: 02.05.2018 JP 2018088911
(71) Applicant: Natias Inc., Kobe-shi, Hyogo 650-0047 (JP)
(72) Inventor: KATAOKA, Masanori, Kobe-shi, Hyogo 650-0047 (JP); HYODO, Mamoru, Kobe-shi, Hyogo 650-0047 (JP)
(74) Representative: Uexküll & Stolberg
(86) International application number: PCT/JP2019/018307
(87) International publication number: WO 2019/212063

(57) **Abstract**

An optically active segment for use in synthesis of a stereocontrolled oligonucleotide represented by the following formula (I), a method for producing the same, and a method for synthesizing a stereocontrolled oligonucleotide therefrom are provided. In formula, B is a protected/unprotected nucleoside base; R¹ is substituted/unsubstituted aliphatic group; R², R³ is a DMTr group or -P(R¹¹)(NR¹²)₂; R¹¹ is OCH₂CH₂CN, SCH₂CH₂CN, etc.; R¹² is a substituted/unsubstituted aliphatic group or aromatic group; X is H, an alkyl, O-alkyl, etc.; Y is H, NHR¹³, a halogen, etc., or a hydroxyl group protected with an acyl, ether, or silyl, or forms an X-Y bond with X; and n is an integer of 0 or more and 4 or less.

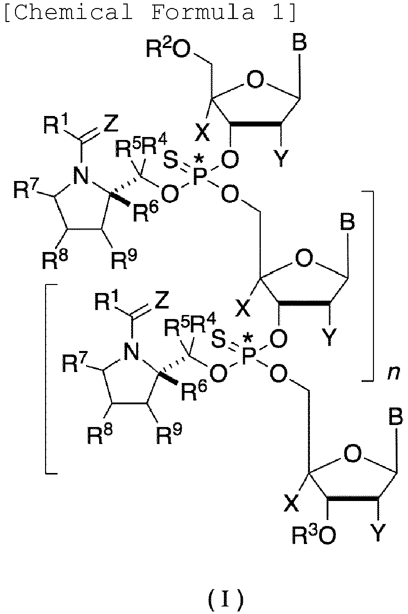

## Description

### [Technical Field]

The present invention relates to an optically active segment for use in synthesis of a stereocontrolled phosphorus atom-modified oligonucleotide, a method for producing the same, and a method for synthesizing a stereocontrolled phosphorus atom-modified oligonucleotide using the optically active segment.

### [Background Art]

In recent years, attention has been focused on nucleic acid drugs having a natural or non-natural oligonucleotide as basic skeleton. Chemical synthesis methods are widely used to obtain nucleic acid drugs designed to obtain an intended effect.

In the case where a thiophosphate oligonucleotide widely used as nucleic acid drug has a thiophosphate in a phosphodiester bond, the thiophosphate oligonucleotide has an asymmetric center on a phosphorus atom that forms a phosphodiester bond due to the presence of a sulfur atom substituting for one of the non-bridging oxygen atoms of the phosphodiester bond.

In a widely used method for synthesizing thiophosphate oligonucleotides, it is difficult to synthesize thiophosphate oligonucleotides having a desired stereochemistry onlyby controlling the configuration on the phosphorus atom. For this reason, in actuality, a diastereomeric mixture is directly used as active pharmaceutical ingredient (API) (refer to Nonpatent Literature 1) .

On the other hand, there are concerns that sides effects are caused by direct administration of the diastereomeric mixture and that an excessive administration of the diastereomeric mixture to the body is required to secure the amount of the thiophosphate having a stereochemistry exhibiting a desired effect.

### [Citation List]

### [Patent Literature]

[PTL 1] PCT International Publication No. WO 2011/108682

### [Non Patent Literature]

[NPL 1] European Medicines Agency, Assessment Report, Spinraza (registered trademark), pp. 13 (2017)

### [Summary of Invention]

### [Technical Problem]

So far, several stereoselective synthesis methods of a thiophosphate oligonucleotide have been attempted. As one of the methods, a synthesis method uses a unit including prolinol as an asymmetric source introduced into the phosphite-binding position of a nucleoside phosphoramidite. In synthesis of an oligonucleotide with a thiophosphate moiety stereocontrolled, however, the method as described in Patent Literature 1 uses a nucleoside monomer-type unit as synthesis unit, so that the monomer-type unit needs to be subjected to step by step condensation.

Specifically, at the stage of finally obtaining an oligonucleotide having a target length, it is necessary to perform a purification step for removing by-products generated in each step as described above and reagent residues. Examples of the typical by-products generated in the method for synthesizing an oligonucleotide N-mer by extending bases one by one include an (N-1)-mer which is shorter by one base and an (N-2)-mer which is shorter by two bases, generated in coupling steps. Such (N-1)-mer and (N-2)-mer are very similar in structure and physical properties to the target N-mer. As a result, in the stage of purifying the N-mer using chromatography or the like, the difference in mobility between the target N-mer and the by-products such as (N-1)-mer and (N-2)-mer is small. For this reason, there exists a problem of a heavy burden imposed by purification for precisely separating the N-mer and others.

Furthermore, in the thiophosphate moiety of a thiophosphate oligonucleotide, a stereoisomer on the phosphorus atom may be mixed. This adds another problem of the burden imposed by purification by which by-products having a different stereochemistry must be separated from a thiophosphate oligonucleotide having the target stereochemistry. Among the synthetic methods known to date, there is no synthetic method that can withstand the practical use of oligonucleotide synthesis with the thiophosphate moiety stereocontrolled to a desired stereochemistry.

In view of such circumstances, it is an object of the present invention to provide a segment for synthesizing an oligonucleotide with a fewer number of steps and reliable stereo control, a method for producing the same, and a method for synthesizing a stereocontrolled oligonucleotide using the same.

### [Solution to Problem]

In order to solve the problems, the optically active segment for use in synthesis of a stereocontrolled oligonucleotide, a method for producing the same, and a method for synthesizing a stereocontrolled oligonucleotide using the same employ the following means.

A first aspect of the present invention relates to an optically active segment for use in synthesis of a stereocontrolled oligonucleotide, represented by the following formula (I).

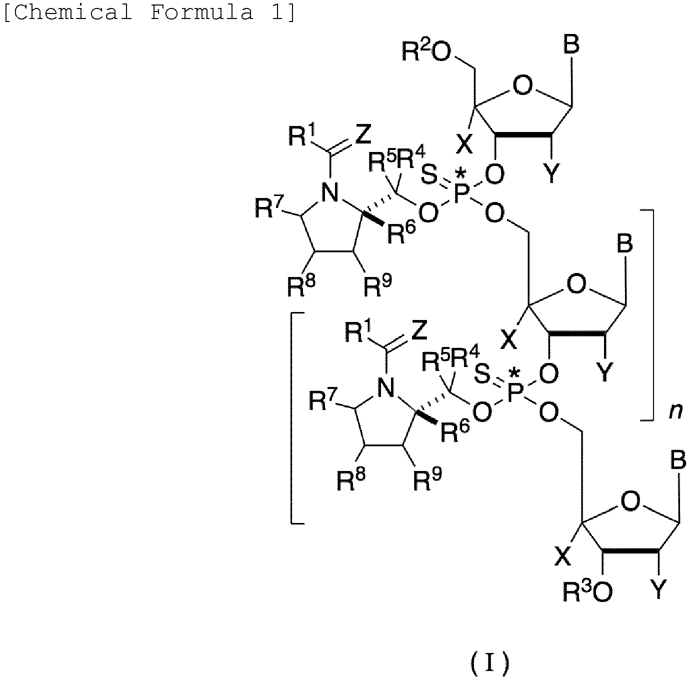

In formula (I), B is independently a nucleoside base unprotected or protected with a protecting group; R¹ is a substituted or unsubstituted aliphatic group, a substituted or unsubstituted aromatic group, or a substituted or unsubstituted heteroaryl group; R³ is -P(R¹¹){N(R¹²)₂} in the case where R² is a protecting group removable under acidic conditions or a silyl protecting group, or R³ is a protecting group removable under acidic conditions or a silyl protecting group in the case where R² is -P(R¹¹){N(R¹²)₂}; R⁴ and R⁵ are independently H, an alkyl, an alkenyl, a substituted or unsubstituted aromatic group, a substituted or unsubstituted heteroaryl group, a -CH₂-substituted or unsubstituted aryl, or a -CH₂-substituted silyl; R⁶, R⁷, R⁸ and R⁹ are independently H, a substituted or unsubstituted aliphatic group, or a substituted or unsubstituted aromatic group; R¹¹ is independently OCH₂CH₂CN, SCH₂CH₂CN, OCH₂CH=CH₂, or OCH₃; R¹² is a substituted or unsubstituted aliphatic group, or a substituted or unsubstituted aromatic group; X is independently H, an alkyl, an O-alkyl, an N-alkyl, or a halogen; Y is independently H, NHR¹³, a halogen, CN, CF₃ or a hydroxyl group protected with an acyl protecting group, an ether protecting group or a silyl protecting group, or forms an X-Y bond with X; R¹³ is independently H, an alkyl, a carbamate, an amide group, or a substituted silyl; Z is independently O or S; and n is an integer of 0 or more and 4 or less.

In the first aspect described above, in the case where B in formula (I) is a nucleoside protected with a protecting group, the protecting group may be an acyl protecting group.

In the first aspect described above, in formula (I), R¹ may be an alkyloxy, methyl, trifluoromethyl, phenyl, or phenylacetyl group, preferably a phenyl group or an acetyl group; X may be H; Y may be preferably H or a hydroxyl group protected with a t-butyldimethylsilyl group; Z may be O; and R¹² may be an isopropyl group. Also, in formula (I), R¹-C(=Z)-may be an acyl protecting group such as an acetyl group, a trifluoroacetyl group, and a benzoyl group.

A second aspect of the present invention relates to a method for producing an optically active segment for use in synthesis of a stereocontrolled oligonucleotide, represented by the following formula (I).

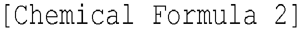

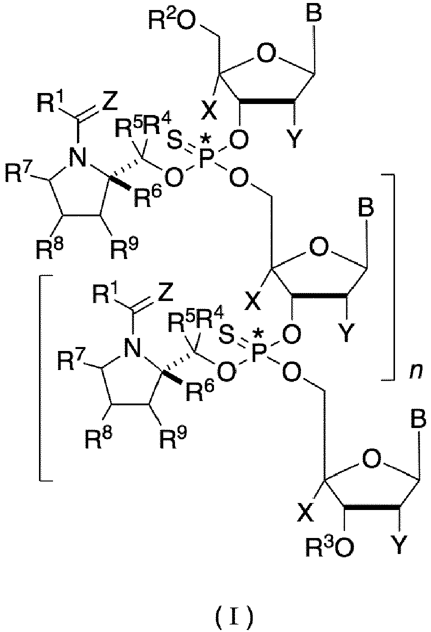

In formula (I), B is independently a nucleoside base unprotected or protected with a protecting group; R¹ is a substituted or unsubstituted aliphatic group, a substituted or unsubstituted aromatic group, or a substituted or unsubstituted heteroaryl group; R³ is -P(R¹¹){N(R¹²)₂} in the case where R² is a protecting group removable under acidic conditions or a silyl protecting group, or R³ is a protecting group removable under acidic conditions or a silyl protecting group in the case where R² is -P(R¹¹){N(R¹²)₂}; R⁴ and R⁵ are independently H, an alkyl, an alkenyl, a substituted or unsubstituted aromatic group, a substituted or unsubstituted heteroaryl group, a -CH₂-substituted or unsubstituted aryl, or a -CH₂-substituted silyl; R⁶, R⁷, R⁸ and R⁹ are independently H, a substituted or unsubstituted aliphatic group, or a substituted or unsubstituted aromatic group; R¹¹ is independently OCH₂CH₂CN, SCH₂CH₂CN, OCH₂CH=CH₂, or OCH₃; R¹² is a substituted or unsubstituted aliphatic group, or a substituted or unsubstituted aromatic group; X is independently H, an alkyl, an O-alkyl, an N-alkyl, or a halogen; Y is independently H, NHR¹³, a halogen, CN, CF₃ or a hydroxyl group protected with an acyl protecting group, an ether protecting group or a silyl protecting group, or forms an X-Y bond with X; R¹³ is independently H, an alkyl, a carbamate, an amide group, or a substituted silyl; Z is independently O or S; and n is an integer of 0 or more and 4 or less.

The production method comprises:
(a) a step of reacting a nucleoside represented by the following formula (II): 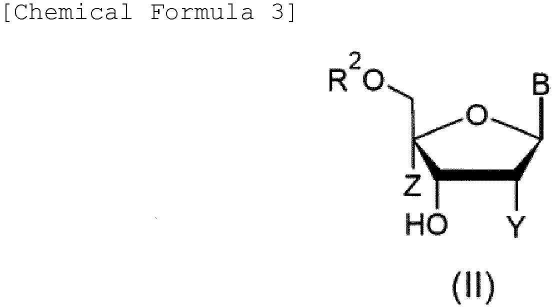 wherein R² is a protecting group removable under acidic conditions or a silyl protecting group,
   with a compound represented by the following formula (III): 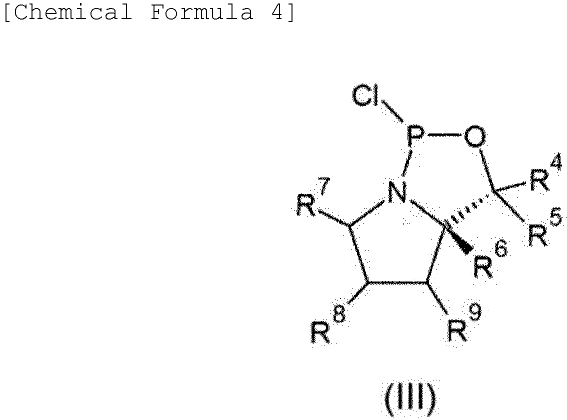 to prepare a compound having a structure represented by the following formula (IV): 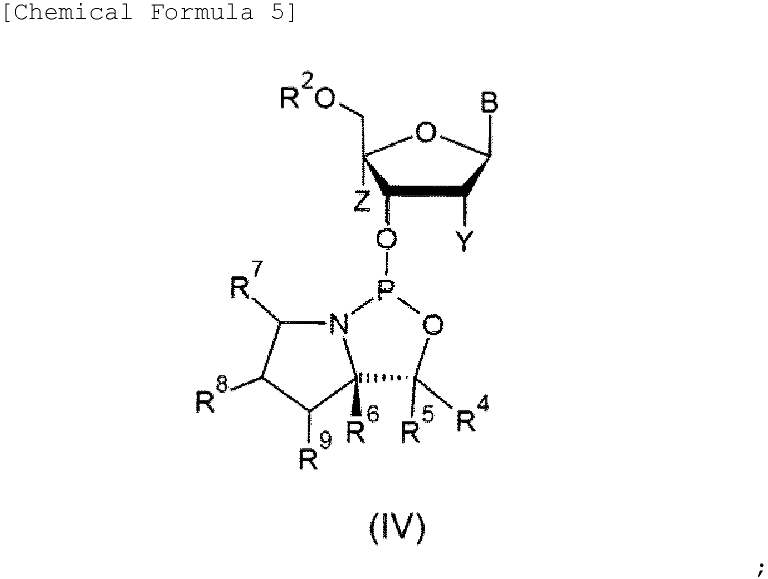
(b) a step of reacting the compound having a structure represented by formula (IV) with a compound having the structure of formula (V): 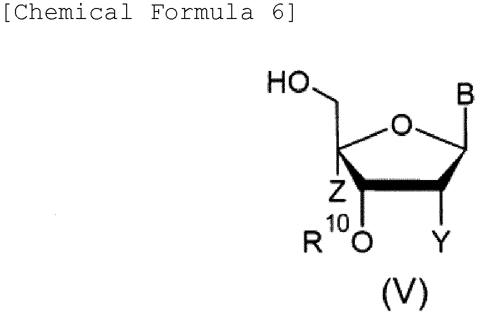 wherein R¹⁰ is an acyl, alkyloxycarbonyl, alkyl, acetal, or silyl protecting group,
   and subsequently performing a sulfurization reaction to prepare a compound having a structure represented by the following formula (VI): 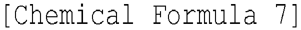 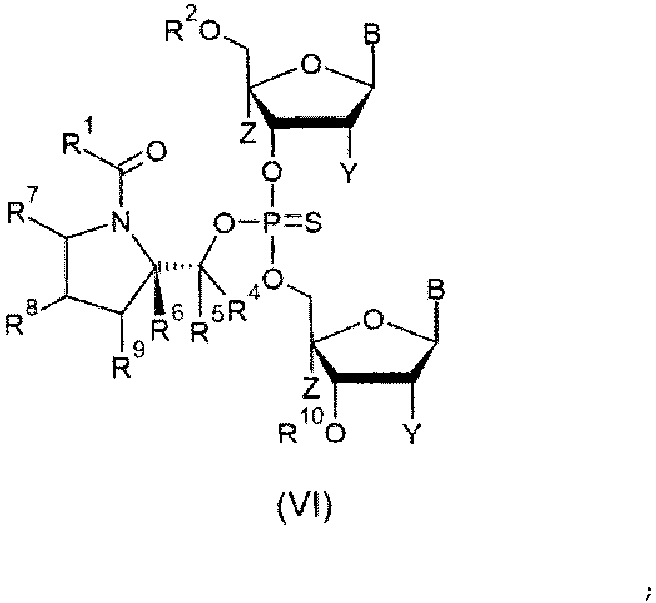
(c) a step of reacting a compound obtained through a deprotection reaction of 5'-hydroxyl group of the compound having the structure of formula (VI) with a compound having the structure of formula (IV) and then performing a sulfurization reaction 1 to 4 times, in the case of n=1 to 4 in formula (I); and
(d) a step of performing a deprotection reaction of the protecting group OR¹⁰ for 3'-hydroxyl group of the compound obtained in the step (b) or (c), and then reacting the product with a phosphitylating compound having a structure of R¹¹P{N(R¹²)₂}₂ to prepare a segment having the structure of formula (I).

In the second aspect, in the case where B in formula (I) is a nucleoside protected with a protecting group, the protecting group may be an acyl protecting group.

In the second aspect, in formula (I), R¹ may be an alkyloxy, methyl, trifluoromethyl, phenyl, or phenylacetyl group, preferably a phenyl group or an acetyl group; X may be H; Y may be preferably H or a hydroxyl group protected with a t-butyldimethylsilyl group; Z may be O, and R¹² may be an isopropyl group. In formula (I), R¹-C(=Z)- may be an acyl protecting group such as an acetyl group, a trifluoroacetyl group, and a benzoyl group.

A third aspect of the present invention relates to a method for synthesizing an oligonucleotide using an optically active segment for use in synthesis of a stereocontrolled oligonucleotide, represented by formula (I).

The method according to the third aspect comprises (a) a condensation step of condensing an amidite moiety of the optically active segment represented by formula (I) with a hydroxyl group of a nucleoside or nucleotide, and (b) a deprotection step of deprotecting the terminal protecting group of the segment for use in synthesis of an oligonucleotide condensed with a nucleoside or nucleotide in the condensation step.

In the third aspect, each of the steps may be performed in a solution.

In the third aspect, each of the steps may be performed on a solid-support.

### [Advantageous Effects of Invention]

According to the optically active segment for use in synthesis of a stereocontrolled oligonucleotide of the present invention, use of an L- or D-prolinol derivative as one of the raw materials as an asymmetric source enables one segment to have a plurality of stereocontrolled thiophosphate groups. Thus, compared with the conventional method in which a stereocontrolled oligonucleotide is synthesized using a nucleoside monomer type unit step by step, the number of steps required for synthesizing a stereocontrolled oligonucleotide having the same length can be reduced.

Further, in the case where a stereocontrolled oligonucleotide is synthesized using an optically active segment for use in synthesis of a stereocontrolled oligonucleotide of the present invention, no by-product having a length of N-1 to N-2 is produced. Furthermore, the optically active segment of the present invention has a very low mixing ratio of by-products having a stereochemistry different from the target stereochemistry on the phosphorus atom of the thiophosphate moiety. For this reason, it is possible to reduce the purification burden of a target N-mer stereocontrolled oligonucleotide, so that the purification can be performed more easily, and a larger amount of the target product can be supplied.

### [Brief Description of Drawing]

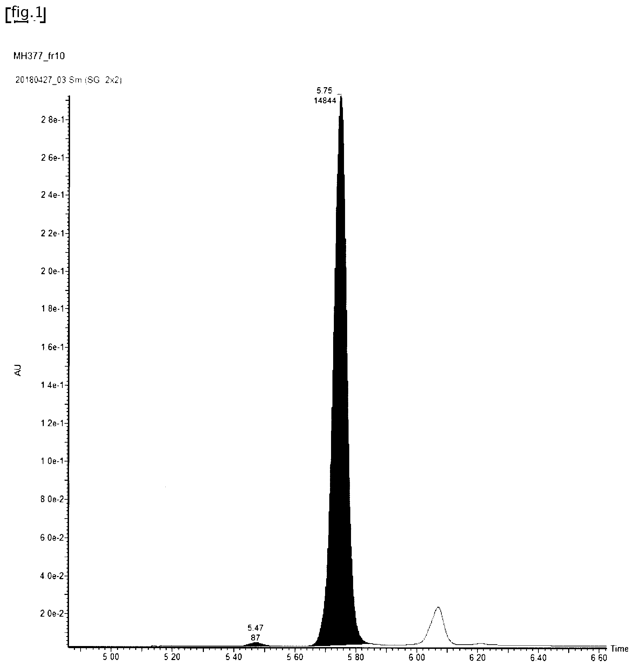

[Fig. 1] Figure 1 is a chart showing a UPLC spectrum of an optically active tetranucleotide obtained in Example 2 in an embodiment of the present invention.

### [Description of Embodiments]

Hereinafter, an embodiment for obtaining an optically active segment for use in synthesis of a stereocontrolled oligonucleotide of the present invention will be described.

An optically active segment for use in synthesis of a stereocontrolled oligonucleotide in the present embodiment has a structure represented by the following formula (I):

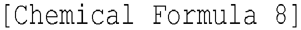

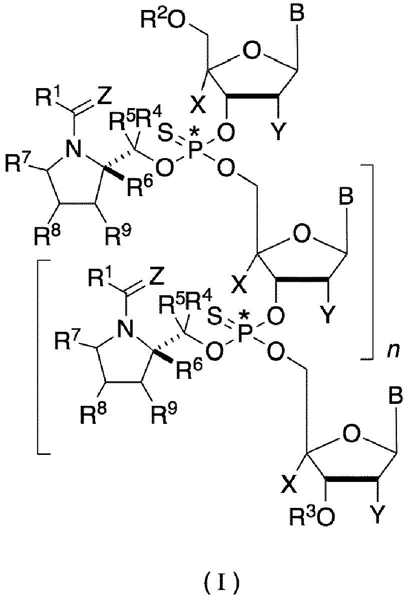

In formula (I), B is independently a nucleoside base unprotected or protected with a protecting group; R¹ is a substituted or unsubstituted aliphatic group, a substituted or unsubstituted aromatic group, or a substituted or unsubstituted heteroaryl group; R³ is -P(R¹¹){N(R¹²)₂} in the case where R² is a protecting group removable under acidic conditions or a silyl protecting group, or R³ is a protecting group removable under acidic conditions or a silyl protecting group in the case where R² is -P(R¹¹){N(R¹²)₂}; R⁴ and R⁵ are independently H, an alkyl, an alkenyl, a substituted or unsubstituted aromatic group, a substituted or unsubstituted heteroaryl group, a -CH₂-substituted or unsubstituted aryl, or a -CH₂-substituted silyl; R⁶, R⁷, R⁸ and R⁹ are independently H, a substituted or unsubstituted aliphatic group, or a substituted or unsubstituted aromatic group; R¹¹ is independently OCH₂CH₂CN, SCH₂CH₂CN, OCH₂CH=CH₂, or OCH₃; R¹² is a substituted or unsubstituted aliphatic group, or a substituted or unsubstituted aromatic group; X is independently H, an alkyl, an O-alkyl, an N-alkyl, or a halogen; Y is independently H, NHR¹³, a halogen, CN, CF₃ or a hydroxyl group protected with an acyl protecting group, an ether protecting group or a silyl protecting group, or forms an X-Y bond with X; R¹³ is independently H, an alkyl, a carbamate, an amide group, or a substituted silyl; Z is independently O or S; and n is an integer of 0 or more and 4 or less.

The optically active segment for use in synthesis of a stereocontrolled oligonucleotide in the present embodiment is synthesized through the following steps of:
(1) synthesizing an optically active phosphorylating agent using an L- or D-prolinol derivative as an asymmetric source,
(2) phosphorylating the 3'-hydroxyl group of a nucleoside having a 5'-hydroxyl group and, on an as needed basis, a nucleoside base moiety, protected with a protecting group, and an unprotected 3'-hydroxyl group (hereinafter referred to as "5'-protected/3'-unprotected nucleoside") using the optically active phosphorylating agent obtained in the step (1) to obtain an optically active 3'-phosphoramidite, and
(3) Performing a reaction between an unprotected 5'-hydroxyl group of the nucleoside having a 3'-hydroxyl group and, on an as needed basis, a nucleoside base moiety, with respective protecting groups (hereinafter referred to as "3'-protected/5'-unprotected nucleoside"), and the optically active 3'-phosphoramidite obtained in the step (2) to obtain a phosphorothioate dimer with a configuration on a phosphorus atom of the phosphate bond controlled to be in an S- or R-form.

Thereafter, in the case of n=0 in a compound represented by formula (I), the protecting group for the 3'-hydroxyl group of the phosphorothioate dimer obtained in the step (3) is deprotected to cause a reaction with a phosphitylatingagent, so that an optically active segment, which is 3'-phosphoramidite of a phosphorothioate dimer with a configuration on a phosphorus atom of the thiophosphate bond controlled to be in an S- or R-form, is synthesized. In the case where n=1 to 4, the protecting group for the 5'-hydroxyl group of the phosphorothioate dimer obtained in the step (3) is deprotected, and the step (3) is repeated as many times as necessary (n times) to synthesize an optically active segment, which is 3'-phosphoramidite of a phosphorothioate (n+1)-mer with a configuration on a phosphorus atom of the phosphate bond controlled to be in an S- or R-form.

By using an acyl, alkyloxycarbonyl, alkyl, acetal, or silyl protecting group as R² of the compound represented by formula (II), and using a protecting group removable under acidic conditions or a silyl protecting group as R⁶ of the compound represented by formula (V), an optically active 5'-phosphoramidite can be synthesized.

According to the optically active segment in the present embodiment, since a configuration on a phosphorus atom of a plurality of thiophosphate bonds is controlled to be in an S- or R-form in a segment, the number of steps required for synthesizing a stereocontrolled oligonucleotide having the same length can be reduced than the conventional method in which a stereocontrolled oligonucleotide is synthesized using a nucleoside monomer type unit step by step. In particular, it is presumed that the optically active segment in the present embodiment functions effectively in synthesis of a nucleic acid drug candidate substance referred to as Gapmer having a plurality of nucleotide phosphorothioates at both ends of an oligonucleotide.

In contrast, although synthesis of a stereocontrolled oligonucleotide using a monomer-type optically active segment has been attempted by other methods as described in Patent Literature 1, synthesis of 3'-phosphoramidite having phosphorothioate dimers or more with a controlled configuration of a plurality of thiophosphate bonds in a segment has not been achieved so far.

Also, in the case where an oligonucleotide with all of the phosphate bond moieties thiophosphated is synthesized using a monomer-type optically active segment, condensation steps are required m times for m phosphate bonds. In contrast, the optically active segment in the present embodiment is subjected to condensation n times in advance, so that a thiophosphated oligonucleotide having the same length can be obtained simply by performing condensation steps m/n times. A highly stereocontrolled oligonucleotides, therefore, can be synthesized in short steps.

The nucleoside base in the present embodiment includes a natural base such as an adenyl group, a guanyl group, a cytosinyl group, a thyminyl group and an uracil group, and a modified base such as a 5-methylcytosinyl group, a 5-fluorouracil group, a 7-methylguanyl group and a 7-deazaadenyl group. The amino group in these nucleoside bases includes a benzyl protecting group, an allyl protecting group, a carbamate protecting group and an acyl protecting group. Preferably, an acyl protecting group such as an acetyl group, a benzoyl group, a phenoxyacetyl group, and an isopropylcarbonyl group is used.

The aliphatic group in the present embodiment includes a saturated or unsaturated, linear or branched C₁-C₁₈ hydrocarbon, and a saturated or unsaturated cyclic C₃-C₁₈ hydrocarbon. A saturated or unsaturated C₁-C₈ hydrocarbon or a cyclic C₃-C₈ hydrocarbon is preferred. The aromatic group in the present embodiment includes a carbocyclic aromatic ring such as a phenyl group, and a carbocyclic aromatic ring condensed with a carbocyclic aromatic ring or a non-carbocyclic aromatic ring such as a naphthyl group. The aliphatic group and the aromatic group in the present embodiment may be substituted with a substituent such as a saturated or unsaturated C₁-C₈ hydrocarbon or C₃-C₈ cyclic hydrocarbon, a halogen, a cyano group, a nitro group, and an aromatic ring.

The protecting groups for 5'-, 3'- or 2'-hydroxyl group in the present embodiment include a protecting group removable under acidic conditions, an acyl protecting group, and a silyl protecting group. The protecting groups removable under acidic conditions include an ether protecting group including a substituted or unsubstituted trityl group and a substituted or unsubstituted tetrahydropyranyl (THP) group, and 4,4'-dimethoxytrityl group is a typical protecting group. The silyl protecting groups include a trimethylsilyl group, a triethylsilyl group, a t-butyldimethylsilyl group, a t-butyldiphenylsilyl group, and a triphenylsilyl group. The acyl protecting groups include an acetyl group and a benzoyl group. Alternatively, a nucleoside with a crosslink bond between the 5'-position and the 2'-position may be used as a raw material. In this case, between the 5'-position and the 2'-position, a bond of (5'-position)-L-O-(2'-position) can be formed, and examples of L include a C₁-C₆ alkylene group, wherein the intermediate carbon atom may be substituted with an oxygen atom or a nitrogen atom to which an alkyl group is bonded.

In the step (1), it is possible to synthesize an optically active phosphorylating agent according to a known synthesis example using a commercially available L- or D-prolinol or an L- or D-prolinol derivative that can be synthesized by a known method as a starting material. In the step (2), the 3'-hydroxy group of a 5'-protected/3'-unprotected nucleoside is reacted with the optically active phosphorylating agent obtained in the step (1) to obtain an optically active 3'-phosphoramidite crude product. The crude product is directly confirmed to be a single stereoisomer.

In the step (2), to a 5'-protected/3'-unprotected nucleoside solution (0.1 to 0.3 M), an optically active phosphorylating agent (1.05 to 2.0 equivalents of 5'-protected/3'-unprotected nucleoside) and a tertiary amine (1.05 to 2.0 equivalents of 5'-protected/3'-unprotected nucleoside) are added at -78°C and then stirred at 0°C for 1 to 2 hours. The optically active 3'-phosphoramidite obtained is used in the next step (3) after purification on silica gel.

In the step (3), to the optically active 3'-phosphoramidite obtained in the step (2), 5'-unprotected/3'-protected nucleoside (0.7 to 0.9 equivalents of 5'-protected/3'-unprotected nucleoside) and an activator (1.05 to 1.2 equivalents of 5'-protected/3'-unprotected nucleoside) are added and reacted at room temperature to obtain a stereocontrolled nucleotide extended by one base unit through purification on silica gel. The yield is about 80 to 95%. In the case where a stereocontrolled nucleotide tetramer or more is synthesized, as an alternative for the method of extending one base each at a time, segments already being a dimer or more may be condensed to each other to achieve extension by two or more base units at a time.

The 5'-protecting group of the resulting stereocontrolled nucleotide is deprotected, and, after purification on silica gel, reacted with a phosphitylating agent to produce a 3'-phosphoramidite. Typical examples of the phosphitylating agent include NCCH₂CH₂OP[N(i-C₃H₇)₂]₂ and CH₂=CHCH₂OP[N(i-C₃H₇)₂]₂, though not limited thereto. Typical examples of the activator include 1H-tetrazole, *S-*ethylthiotetrazole, dicyanoimidazole, and a salt of sulfonic acid and azole or a tertiary amine, though not limited thereto. On an as needed basis, the step (3) is further performed to obtain an optically active 3'-phosphoramidite tetramer or more.

Synthesis of an oligonucleotide using the segment for use in synthesis of oligonucleotide represented by formula (I) may be performed in a solution (hereinafter referred to as "liquid-phase synthesis method"), or may be performed on a solid-support (hereinafter referred to as "solid-phase synthesis method"). In the case where the synthesis is performed by the liquid-phase synthesis method, the 3'-protected/5'-unprotected nucleoside having a hydroxyl group at the 3' end to which a silyl protecting group or an aliphatic- -containing protecting group introduced to increase the solubility in the reaction solvent is used to be subjected to repetition of a condensation step (a) of condensation with an optically active segment, an oxidation step (b), and a deprotection step (c). In the case where the synthesis is performed by the solid-phase synthesis method, a condensation step (a) of condensation with an optically active segment, a capping step (b), an oxidation step (c), and a deprotection step (d) are repeated. In both of the methods, a target oligonucleotide can be obtained through a subsequent deprotection treatment under basic conditions. On this occasion, by using phenylacetyl disulfide (PADS) as a sulfurizing agent, a pyrrolidine moiety can be protected at the same time, so that the capping step may be omitted.

In either case of using the liquid-phase synthesis method and the solid-phase synthesis method, as the first step of oligonucleotide synthesis, a step of activating the 3'-terminal amidite of a compound represented by formula (I) with an activator so as to be condensed with a 3'-protected/5'-unprotected nucleoside or nucleotide is performed. As the activator, a commonly used phosphite activator may be used, and examples thereof include 1*H*-tetrazole, *S-*ethylthiotetrazole, dicyanoimidazole, and a salt of sulfonic acid and azole or a tertiary amine, though not limited thereto. The time required for the coupling reaction is generally about 1 minute to 30 minutes, depending on the scale of the reaction.

Next, as a second step in synthesis of a stereocontrolled oligonucleotide, an oxidation step of reacting the intermediate obtained in the condensation step with an oxidizing agent to obtain a phosphate nucleotide is performed.

Subsequently, as a third step in the oligonucleotide synthesis, the intermediate obtained in the oxidation step is reacted with an anhydrous acidic solution to obtain a 5'-hydroxyl unprotected nucleotide.

In the stereocontrolled oligonucleotide synthesized by using the optically active segment for use in synthesis of a stereocontrolled oligonucleotide in the present embodiment, a protecting group for the nucleoside base, a protecting group for the 5'-, 3'- or 2'-hydroxyl group, and a protecting group for phosphoric acid in the phosphate bond are deprotected under deprotection conditions corresponding to the protecting group used. Thereby, a target stereocontrolled oligonucleotide is obtained.

The following Examples illustrate an embodiment of the present invention. According to the procedure shown in Example 1, an optically active 3'-phosphoramidite trimer which is an example of the compound represented by formula (I) was produced. Also, according to the procedure shown in Example 2, a tetranucleotide having an optically active phosphoric acid moiety was produced through an optically active 3'-phosphoramidite which is an example of the compound represented by formula (I). Further, according to the procedure shown in Example 3, synthesis of a stereocontrolled oligonucleotide using an optically active 3'-phosphoramidite which is one of the compounds represented by formula (I) can be performed.

### {Example 1}

### (Step 1: Synthesis of optically active phosphorylating agent)

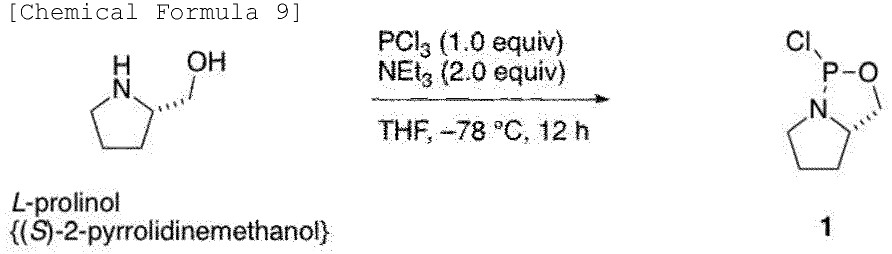

Phosphorus trichloride (3.5 mL, 5.5 g, 40 mmol) was dissolved in toluene (50 mL) and brought to a temperature of -78°C. Separately, L-prolinol (3.9 mL, 4.0 g, 40 mmol) and triethylamine (12 mL, 8.9 g, 88 mmol) were dissolved in toluene (50 mL) and added dropwise to phosphorus trichloride over 1 hour. The mixture was stirred as it was for 12 hours, and then returned to a temperature of 0°C so as to be further stirred for 1 hour. After the reaction, a by-produced precipitate was removed by celite filtration, and the solvent was distilled off under reduced pressure to obtain a crude product. This was distilled under reduced pressure (65°C, 1.0 mmHg) to obtain a target phosphorochloridite 1 (3.2 g, 19 mmol, 48% yield).

### (Step 2: Synthesis of optically active phosphoramidite 2)

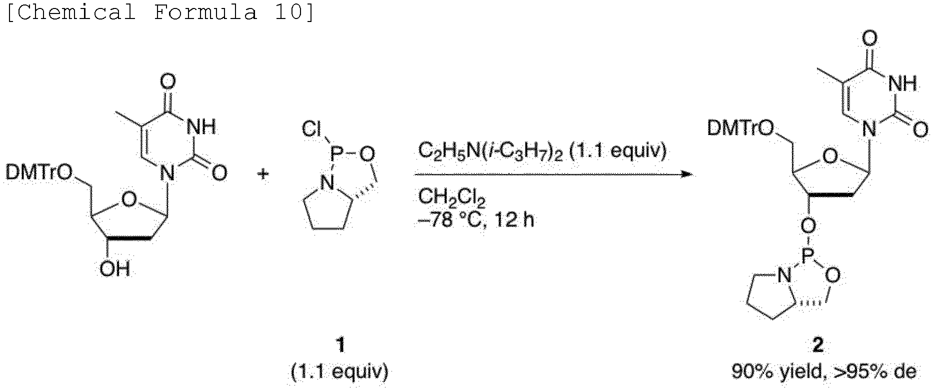

5'-DMTr protected thymidine (11 g, 21 mmol, manufactured by Hongene Biotech Corporation) was dissolved in dichloromethane (150 mL), to which diisopropylethylamine (3.9 mL, 3.0 g, 23 mmol) was added, and the mixture was cooled to a temperature of -78°C. Separately, phosphorochloridite 1 (3.8 g, 23 mmol) was dissolved in dichloromethane (50 mL) and added dropwise to the thymidine solution over 30 minutes. After raising the temperature to 0°C over 12 hours, the mixture was stirred at 0°C for 1 hour to complete the reaction. The reaction mixture obtained was washed with a saturated aqueous solution of sodium hydrogen carbonate and saturated brine, dried over sodium sulfate, and the solvent was distilled away to obtain a target optically active phosphoramidite 2 (16 g) as a crude product. The product was presumed to be almost a signal stereoisomer, resulting from observation of one signal at 154 ppm in ³¹PNMR measurement.

### (Step 3: Synthesis of 5'-unprotected nucleoside)

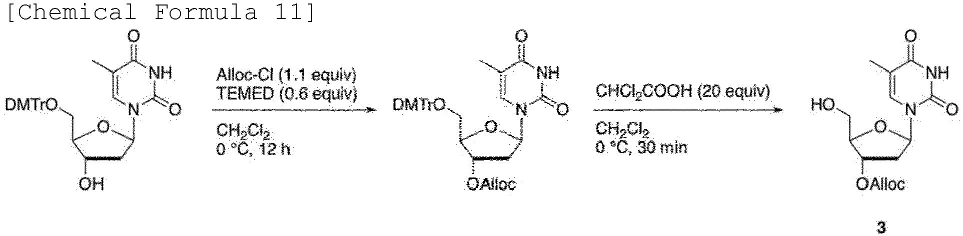

5'-DMTr protected thymidine (2.7 g, 5.0 mmol, manufactured by Hongene Biotech Corporation) was dissolved in dichloromethane (50 mL) and brought to a temperature of 0°C. Tetramethyl ethylenediamine (450 µL, 350 mg, 3.0 mmol) and allyloxycarbonyl chloride (590 µL, 650 mg, 5.5 mmol) were added thereto to initiate a reaction. After 12 hours, the reaction mixture was partitioned between dichloromethane (100 mL) and saturated aqueous solution of sodium hydrogen carbonate (100 mL), and an organic layer was collected. The organic layer was washed with saturated brine (50 mL) and dried over sodium sulfate to obtain a crude product. The product was purified by column chromatography using hexane-ethyl acetate as an elution solvent to obtain a target 5'-DMTr-3'-Alloc thymidine (3.0 g, 4.8 mmol, 95% yield). ESI-MS: 651.5 [(M+Na)⁺]

The compound was dissolved in dichloromethane (50 mL), cooled to 0°C, and a dichloroacetic acid (8.3 mL, 13 g, 100 mmol)/dichloromethane (50 mL) solution was added thereto to initiate a reaction. After confirming that the reaction mixture was colored in red to indicate the release of a trityl cation, the reaction mixture was directly subjected to column chromatography using ethyl acetate-methanol as an elution solvent to obtain a target product 3 (1.3 g, 4.0 mmol, 80% yield). ESI-MS: 340.9[(M+Na)⁺].

### (Step 4: Synthesis of dinucleotide having optically active thiophosphate bond)

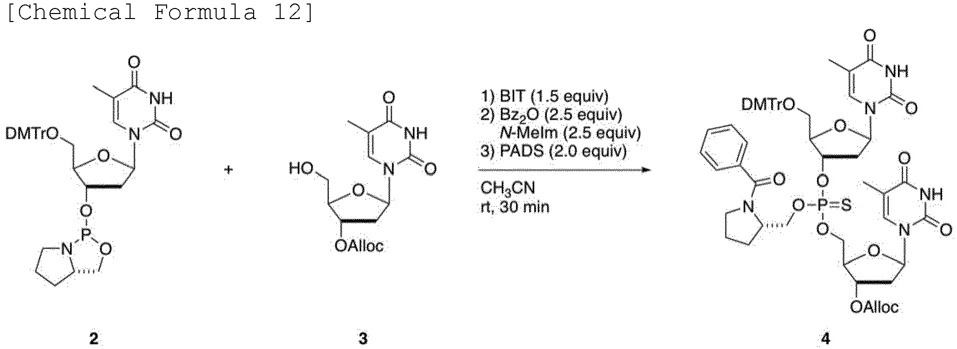

Optically active phosphoramidite 2 (2.7 g, 4.0 mmol) and 5'-hydroxyl unprotected nucleoside 3 (1.0 g, 3.1 mmol) were dissolved in acetonitrile (20 mL), and benzoimidazolium triflate (1.2 g, 4.6 mmol) was added thereto. After 30 minutes, N-methylimidazole (0.49 mL, 510 mg, 6.2 mmol) was added and then benzoic anhydride (1.7 g, 7.7 mmol) was added. The mixture was further stirred for 30 minutes. Finally, phenylacetyl disulfide (1.9 g, 6.2 mmol) was added and stirred for 30 minutes. The reaction mixture was partitioned between dichloromethane (100 mL) and saturated aqueous solution of sodium hydrogen carbonate (100 mL), and an organic layer was collected. The organic layer was washed with saturated brine (50 mL) and dried over sodium sulfate to obtain a crude product. The product was purified by column chromatography using hexane-ethyl acetate-methanol as an elution solvent to obtain a target compound 4 (3.2 g, 2.8 mmol, 91% yield) having an optically active thiophosphate bond. ESI-MS: 1159.1 [(M+Na)⁺].

### (Step 5: Deprotection of 5' protecting group of optically active phosphoric acid moiety of dinucleotide)

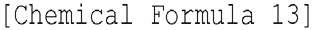

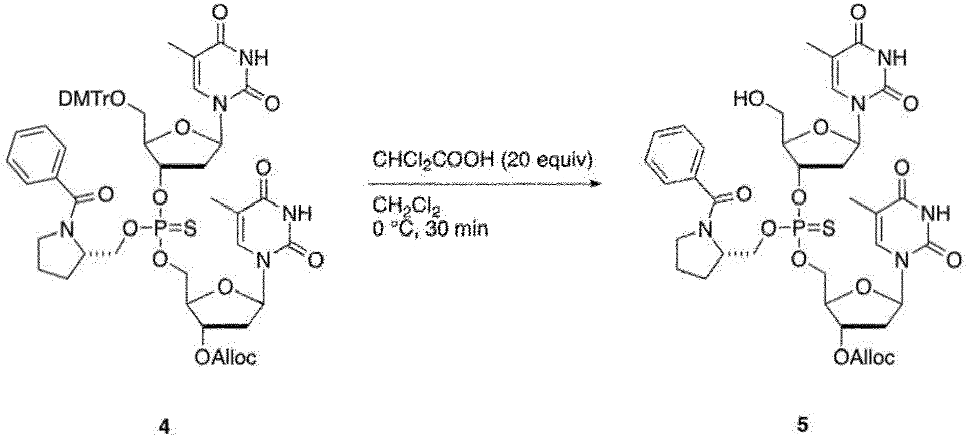

The optically active dinucleotide 4 (3.2 g, 2.8 mmol) was dissolved in dichloromethane (28 mL) and brought to a temperature of 0°C. Dichloroacetic acid (4.6 mL, 7.2 g, 56 mmol) dissolved in dichloromethane (24 mL) was slowly added thereto, and after confirming that the reaction mixture was colored in red to indicate the release of trityl cations, the mixture was stirred for 30 minutes and then the reaction mixture was directly subjected to column chromatography to obtain a target compound 5 (1.9 g, 2.2 mmol, 79% yield).

### (Step 6: Synthesis of optically active trinucleotide)

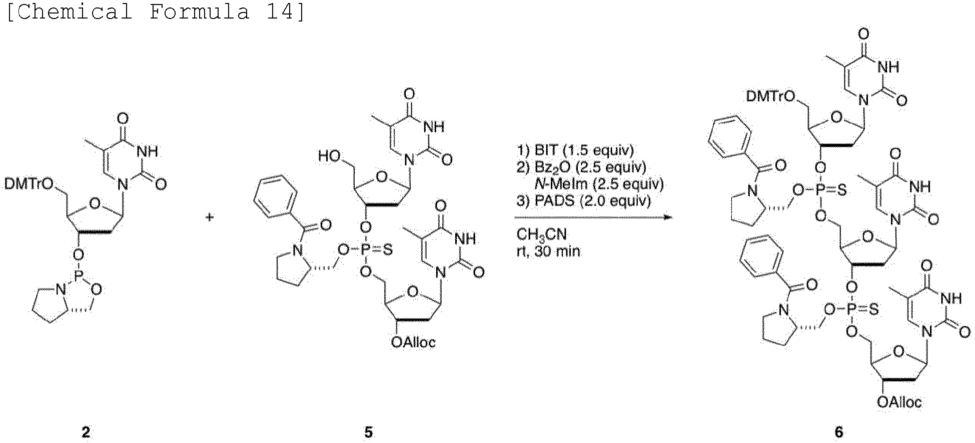

The optically active phosphoramidite 2 (1.9 g, 2.9 mmol) and the 5'-hydroxyl unprotected dinucleotide 5 (1.8 g, 2.2 mmol) were dissolved in acetonitrile (14 mL), and benzoimidazolium triflate (870 mg, 3.2 mmol) was added thereto. After 30 minutes, N-methylimidazole (0.35 mL, 340 mg, 4.3 mmol) was added, and then benzoic anhydride (1.2 g, 5.4 mmol) was added. The mixture was further stirred for 30 minutes. Finally, phenylacetyl disulfide (1.3 g, 4.3 mmol) was added and stirred for 30 minutes. The reaction mixture was partitioned between dichloromethane (100 mL) and a saturated aqueous solution of sodium hydrogen carbonate (100 mL), and an organic layer was collected. The organic layer was washed with saturated brine (50 mL) and dried over sodium sulfate to obtain a crude product. The product was purified by column chromatography using hexane-ethyl acetate-methanol as an elution solvent to obtain a target compound 6 (2.9 g, 1.8 mmol, 83% yield).

### (Step 7: Deprotection of 3'-hydroxyl protecting group of optically active trinucleotide)

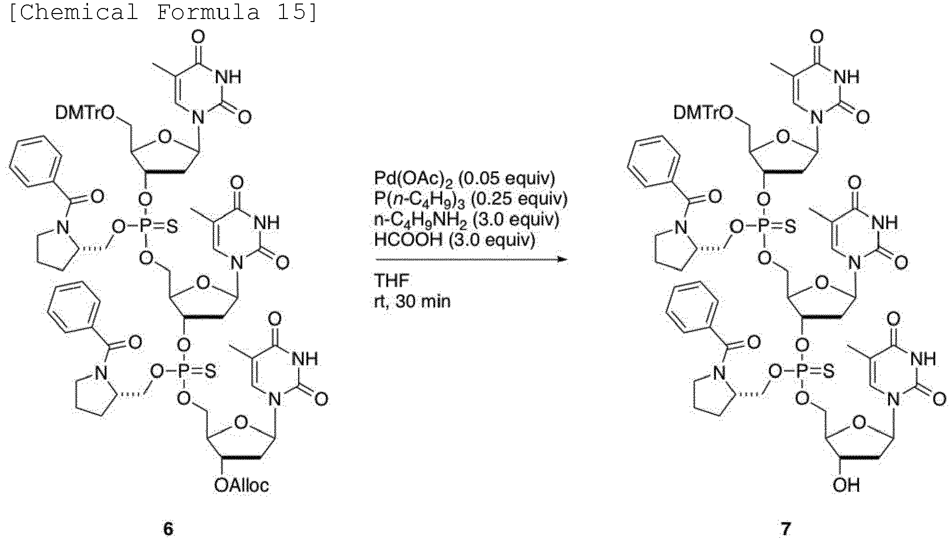

The optically active trinucleotide 6 (330 mg, 0.2 mmol) was dissolved in tetrahydrofuran (5 mL), and triphenylphosphine (20 mg, 0.1 mmol), butylamine (23 µL, 0.6 mmol), formic acid (60 µL, 0.6 mmol), and palladium acetate (4.5 mg, 0.02 mmol) were sequentially added thereto to initiate the reaction. After 6 hours, the solvent was distilled off, and the product was dissolved in dichloromethane. After celite filtration of the solution, the resulting crude product was subjected to column chromatography. As a result, a 3'-unprotected trinucleotide 7 (280 mg, 0.18 mmol, 90% yield) was obtained.

### (Step 8: Synthesis of optically active trinucleotide phosphoramidite)

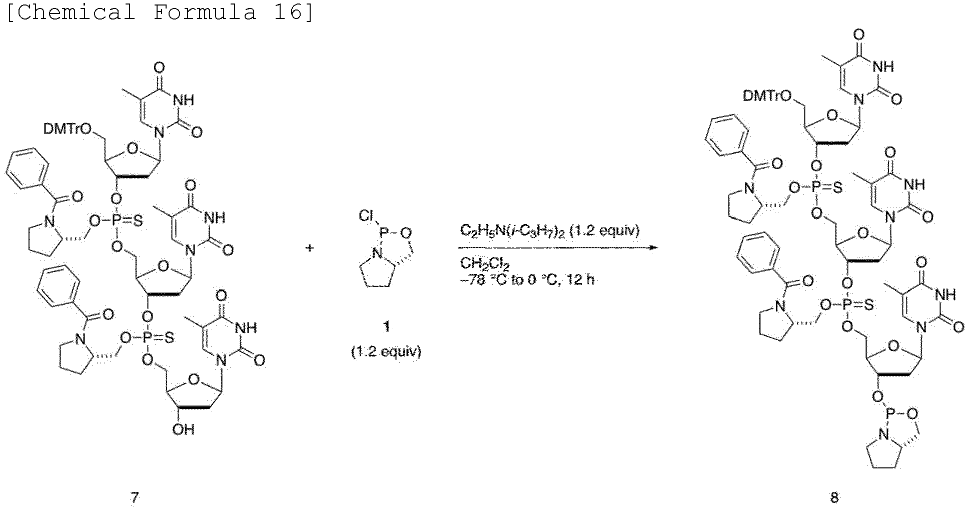

The 3'-unprotected trinucleotide 7 (280 mg, 0.18 mmol) was dissolved in dichloromethane (9 mL), to which diisopropylethylamine (37 µL, 28 mg, 0.22 mmol) was added, and the mixture was cooled to a temperature of -78°C. Separately, the compound 1 (36 mg, 0.22 mmol) was dissolved in dichloromethane (9 mL) and added dropwise to the reaction solution over 30 minutes. After raising the temperature to 0°C over 12 hours, the mixture was stirred at 0°C for 1 hour to complete the reaction. The reaction mixture obtained was washed with a saturated aqueous solution of sodium hydrogen carbonate and saturated brine, and dried over sodium sulfate.

The solvent was distilled to obtain a target trinucleotide amidite 8 (230 mg, 0.14 mmol, 60% yield) . From the ³¹PNMR measurement, formation of the target product was suggested.

### {Example 2}

### (Sep 9: Synthesis of optically active dinucleotide (N-acetyl capping))

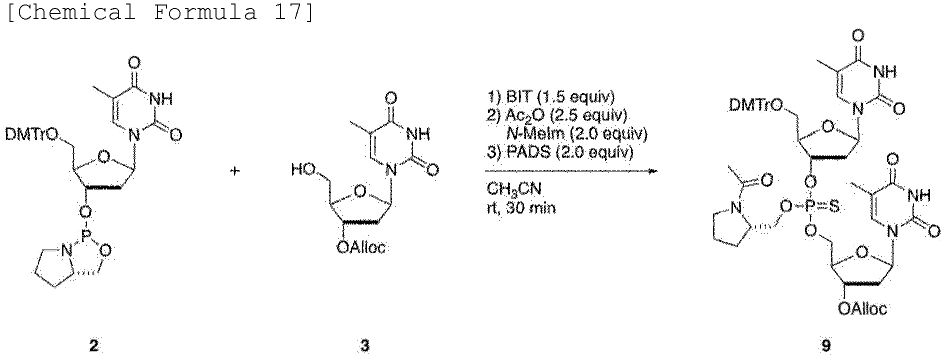

The optically active phosphoramidite 2 (2.7 g, 4.0 mmol) and the 5'-hydroxyl unprotected nucleoside 3 (1.0 g, 3.1 mmol) were dissolved in acetonitrile (20 mL), and benzoimidazolium triflate (1.2 g, 4.6 mmol) was added thereto. After 30 minutes, N-methylimidazole (0.49 mL, 510 mg, 6.2 mmol) was added, and subsequently acetic anhydride (0.73 mL, 790 mg, 7.7 mmol) was added thereto. The mixture was further stirred for 30 minutes. Finally, phenylacetyl disulfide (1.9 g, 6.2 mmol) was added and stirred for 30 minutes. The reaction mixture was partitioned between dichloromethane (100 mL) and saturated aqueous solution of sodium hydrogen carbonate (100 mL), and an organic layer was collected. The organic layer was washed with saturated brine (50 mL) and dried over sodium sulfate to obtain a crude product. The product was purified by column chromatography using hexane-ethyl acetate-methanol as an elution solvent to obtain a target compound 9 (2.4 g, 2.2 mmol, 72% yield). ESI-MS: 1096.8 [(M+Na)⁺].

### (Step 10: Deprotection of 5'-protecting group of optically active phosphoric acid moiety of dinucleotide)

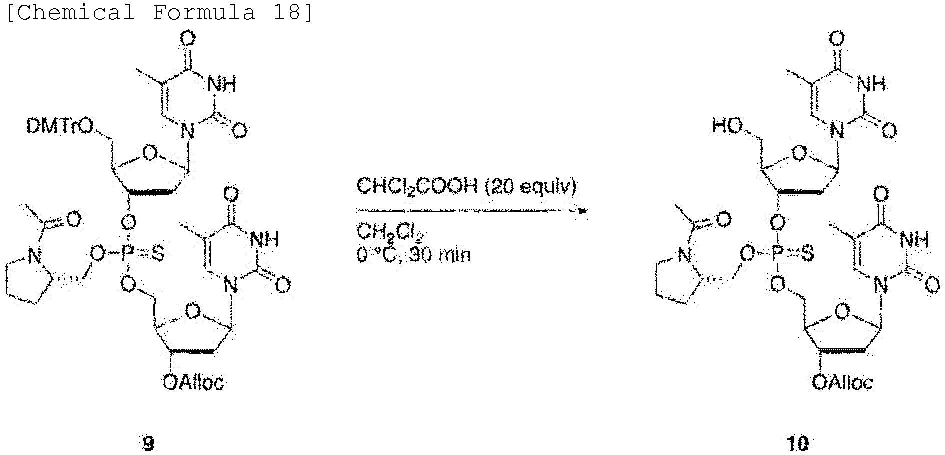

The optically active dinucleotide 9 (780 mg, 0.73 mmol) obtained in Step 9 was dissolved in dichloromethane (7.3 mL) and brought to a temperature of 0°C. Dichloroacetic acid (1.2 mL, 1.9 g, 14 mmol) dissolved in dichloromethane (6.1 mL) was slowly added thereto, and after confirming that the reaction mixture was colored in red indicating the release of trityl cations. After stirring for 30 minutes, the reaction mixture was directly subjected to column chromatography to obtain a target compound 10 (500 mg, 0.65 mmol, 89% yield). ESI-MS: 794.6 [(M+Na)⁺].

### (Step 11: Deprotection of 3'-hydroxyl protecting group of optically active dinucleotide)

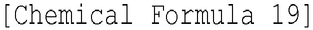

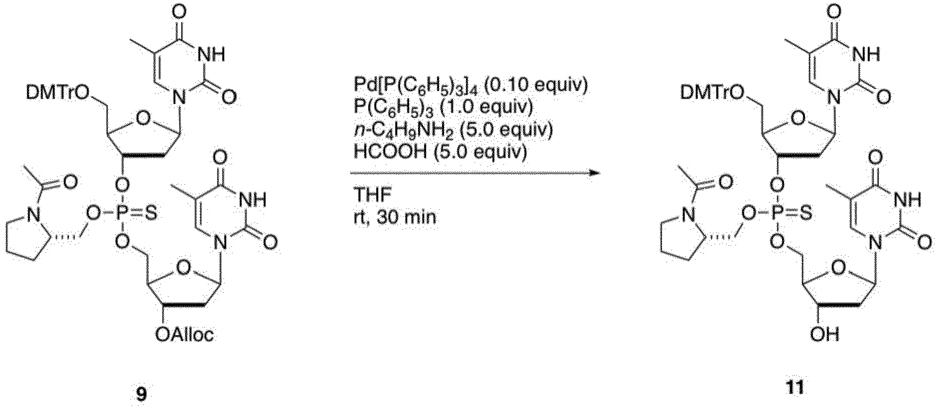

The optically active dinucleotide 9 (1.5 g, 1.4 mmol) was dissolved in tetrahydrofuran (15 mL), to which triphenylphosphine (370 mg, 1.4 mmol), butylamine (700 µL, 7.0 mmol), formic acid (260 µL, 7. 0 mmol), and tetrakis triphenylphosphine palladium (81 mg, 0.07 mmol) were sequentially added to initiate a reaction. After 17 hours, the solvent was distilled off, and a product was dissolved in dichloromethane. After celite filtration of the solution, the resulting crude product was subjected to column chromatography to obtain a 3'-unprotected dinucleotide 11 (1.1 g, 1.1 mmol, 79% yield). ESI-MS: 1012.7 [(M+Na)⁺].

### (Step 12: Synthesis of optically active dinucleotide phosphoramidite)

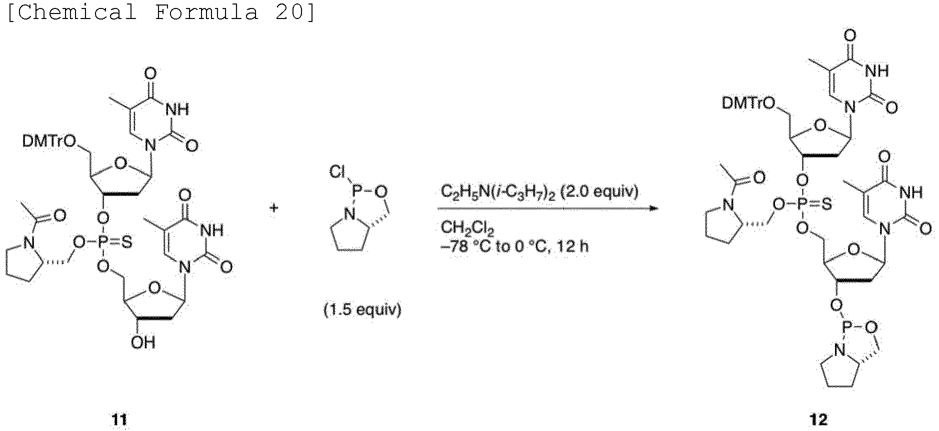

The dinucleotide 11 (990 mg, 1.0 mmol) was dissolved in dichloromethane (10 mL), to which diisopropylethylamine (340 µL, 260 mg, 2.0 mmol) was added, and the mixture was cooled to a temperature of -78°C. Separately, the phosphorochloridite 1 (250 mg, 1.5 mmol) was dissolved in dichloromethane (10 mL) and added dropwise to the reaction solution over 30 minutes. After heating up to 0°C over 12 hours, the solution was stirred at 0°C for 1 hour and then the reaction was completed. The reaction mixture obtained was washed with saturated aqueous solution of sodium hydrogen carbonate and saturated brine and dried over sodium sulfate. The solvent was distilled off to obtain an optically active dinucleotide phosphoramidite 12 (1.2 g) as target product. From ³¹PNMR measurement, the formation of the target product was suggested.

### (Step 13: Synthesis of optically active tetranucleotide by condensation of segments to each other)

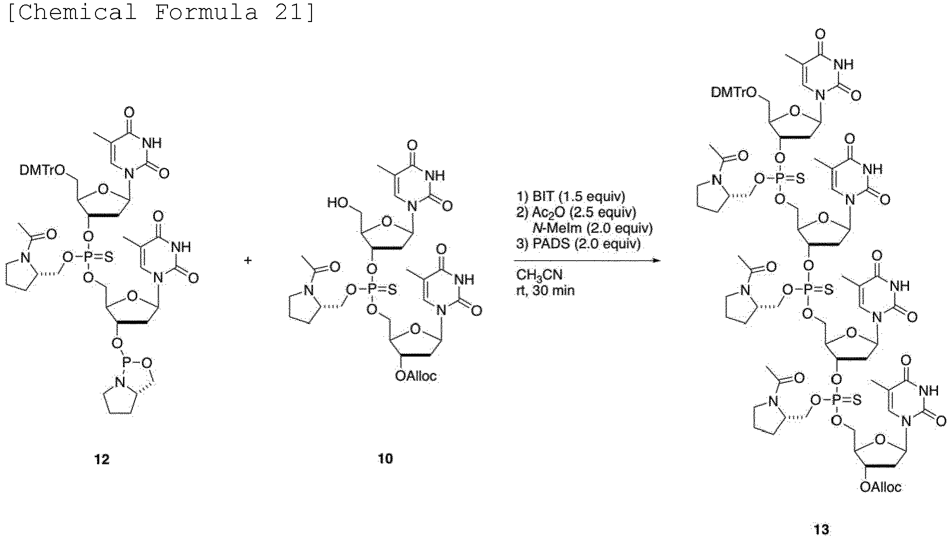

The optically active dinucleotide phosphoramidite 12 (900 mg, 0.8 mmol) and 5'-hydroxyl unprotected dinucleotide 10 (480 mg, 0.62 mmol) were dissolved in acetonitrile (8 mL) and dichloromethane (4 mL), and benzoimidazolium triflate (250 mg, 0.92 mmol) was added therein. After 30 minutes, N-methylimidazole (0.097 mL, 100 mg, 1.2 mmol) was added, and subsequently acetic anhydride (0.15 mL, 160 mg, 1.5 mmol) was added. The mixture was further stirred for 30 minutes. Finally, phenylacetyl disulfide (370 mg, 1.2 mmol) was added and stirred for 30 minutes. The reaction mixture was partitioned between dichloromethane (100 mL) and saturated aqueous solution of sodium hydrogen carbonate (100 mL), and an organic layer was collected. The organic layer was washed with saturated brine (50 mL) and dried over sodium sulfate to obtain a crude product. The product was purified by column chromatography using hexane-ethyl acetate-methanol as an elution solvent to obtain a target compound 13 (1.0 g, 0.51 mmol, 63% yield). ESI-MS: 1988.0 [(M+Na)⁺].

### (Step 14: Deprotection of 5'-protecting group of tetranucleotide having optically active phosphorus atoms)

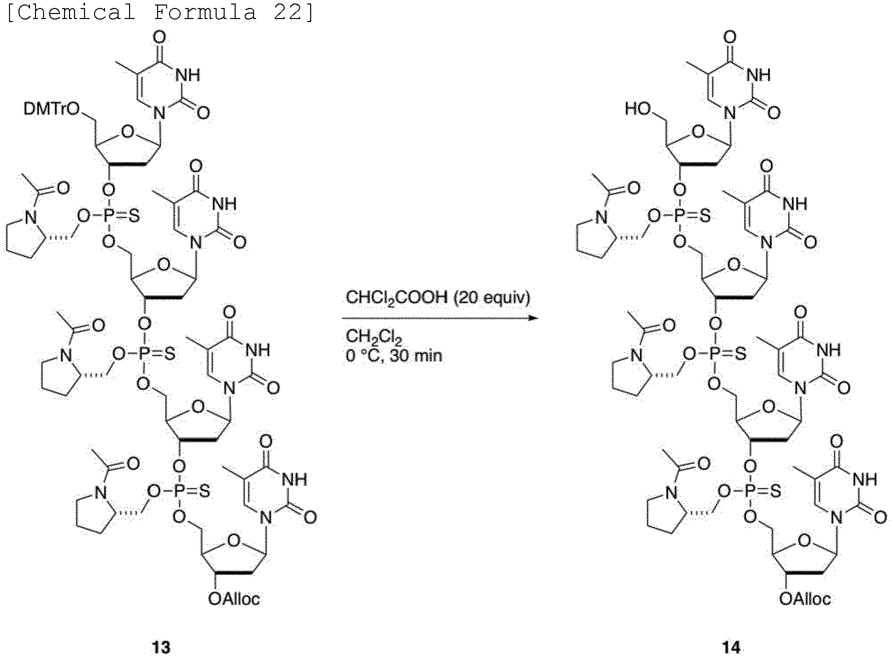

The optically active tetranucleotide 13 (980 mg, 0.50 mmol) was dissolved in dichloromethane (10 mL) and brought to a temperature of 0°C. Dichloroacetic acid (0.83 mL, 1.3 g, 10 mmol) dissolved in dichloromethane (9.2 mL) was slowly added thereto, and after confirming that the reaction mixture was colored in red indicating the release of trityl cations, the solution was stirred for 30 minutes. The reaction mixture was then directly subjected to column chromatography to obtain a target compound 14 (380 mg, 0.23 mmol, 46% yield). ESI-MS: 1684.6 [(M+Na)⁺].

### (Step 15: Synthesis of tetranucleotide having optically active phosphorus atoms)

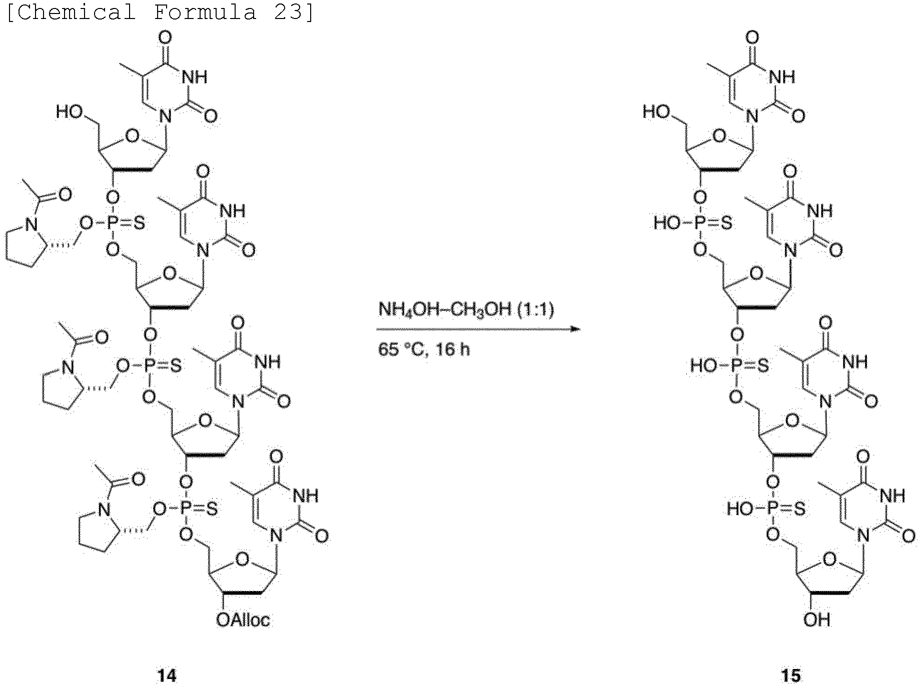

The optically active tetranucleotide 14 having a protected 3'-hydroxyl group (31 mg, 0.019 mmol) was dissolved in methanol (0.5 mL) and 28% aqueous ammonia (0.5 mL), and brought to a temperature of 65°C. After heating for 16 hours, the solution was subjected to centrifugal concentration to distill off ammonia. The product was dissolved in pure water and subjected to reverse phase preparative column chromatography to obtain a target compound 15 (3.8 mg, 0.003 mmol, 17% yield). ESI-MS: 1201 [(M-H)⁻].

In Figure 1, a UPLC-MS spectrum of the resulting nucleotide tetramer having optically active phosphorus atoms 15 is shown. The main absorption that appeared at 5.75 minutes showed a molecular ion peak 1201 in mass spectrometry, so that the target product 15 was identified. Also, the absorption that appeared at 6.07 minutes after appearance of the target product showed a molecular ion peak 1244 in mass spectrometry, so that a tetramer with insufficient deprotection was identified. In contrast, the absorption that appeared at 5.47 minutes was a molecular ion peak 1201 similar to that of the target product in mass spectrometry, suggesting the identification of a diastereomer, and no other absorption was observed. It was therefore presumed that the diastereomeric by-product in the present method was identified only at the absorption appeared at 5.47 minutes. From the ratio of the integral value of the main absorption appearing at 5.75 minutes to the absorption appearing at 5.47 minutes, the excess diastereomer ratio of the resulting nucleotide tetramer having an optically active phosphoric acid moiety 15 was found to be 98.8%.

### {Example 3}

### (Synthesis of oligonucleotide)

In an oligonucleotide solid-phase synthesizer, a stereocontrolled oligonucleotide is synthesized using the optically active phosphoramidite obtained in the present embodiment. After performing a condensation reaction, a capping reaction, and on an as needed basis, an oxidation or sulfurization reaction, according to the standard protocol of the synthesizer, the solid-support is taken out, and the resulting oligonucleotide is detached from the solid-support and subjected to deprotection using concentrated aqueous ammonia.

From the above, the optically active segment for synthesis of a stereocontrolled oligonucleotide in the present embodiment can have not only one but also a plurality of stereocontrolled thiophosphate groups in one segment, using a raw material L- or D-prolinol derivative as an asymmetric source. For this reason, compared with the conventional method in which a stereocontrolled oligonucleotide is synthesized using a nucleoside monomer type unit by step by step, the number of steps required for synthesizing a stereocontrolled oligonucleotide having the same length can be reduced. In the case where a stereocontrolled nucleotide tetramer or more is synthesized, as an alternative for the method of extending one base each at a time, segments already being dimers or more may be condensed to each other to extend two or more base units at a time.

Also, the optically active segment for synthesis of a stereocontrolled oligonucleotide in the present embodiment has a thiophosphate moiety stereocontrolled in advance. For this reason, it is possible to synthesize an oligonucleotide having a desired stereochemistry by using the optically active segment of the present invention only for a moiety to be stereocontrolled and using a commercially available phosphoramidite for other moieties.

Further, the segment for use in synthesis of an oligonucleotide in the present embodiment enables to reduce the number of steps required for synthesizing the same N-mer oligonucleotide in comparison with the conventional method of extending one base each at a time. The yield of the stereocontrolled oligonucleotide having a target length can be therefore improved.

Further, the optically active segment for use in synthesis of a stereocontrolled oligonucleotide in the present embodiment may be effectively used not only in the case where a large amount of stereocontrolled oligonucleotide having a relatively short chain is synthesized by a liquid phase synthesis method, but also in the case where a part of an oligonucleotide having a long chain is stereocontrolled in a solid phase synthesis, for example, in Gapmer synthesis which has been attracting attention in recent years. The load for separating stereoisomers after synthesis of an oligonucleotide can be therefore greatly reduced, and particularly the purification load after synthesis of an N-mer oligonucleotide having a long chain can be reduced by a more convenient purification to obtain a reliably stereocontrolled oligonucleotide.

## Claims

1. An optically active segment for use in synthesis of a stereocontrolled oligonucleotide, represented by the following formula (I): 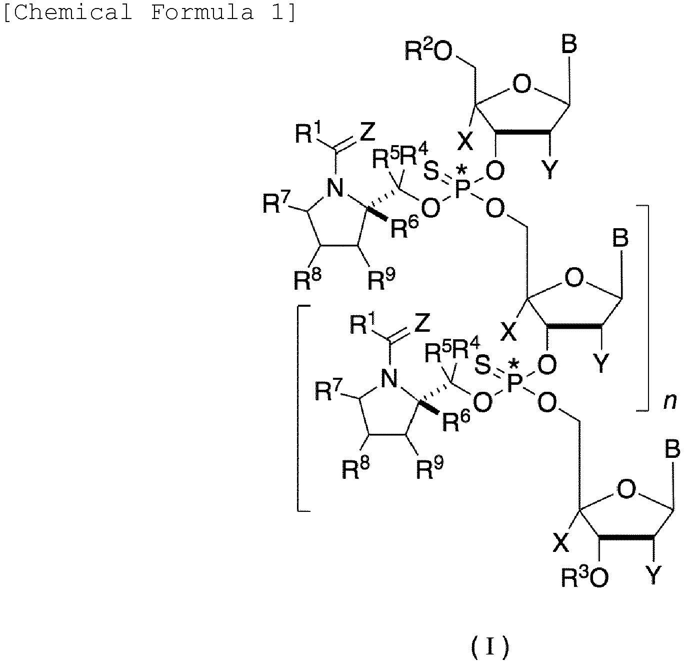
wherein B is independently a nucleoside base unprotected or protected with a protecting group;
R¹ is a substituted or unsubstituted aliphatic group, a substituted or unsubstituted aromatic group, or a substituted or unsubstituted heteroaryl group;
R³ is -P(R¹¹){N(R¹²)₂} in the case where R² is a protecting group removable under acidic conditions or a silyl protecting group, or R³ is a protecting group removable under acidic conditions or a silyl protecting group in the case where R² is -P(R¹¹){N(R¹²)₂};
R⁴ and R⁵ are independently H, an alkyl, an alkenyl, a substituted or unsubstituted aromatic group, a substituted or unsubstituted heteroaryl group, a -CH₂-substituted or unsubstituted aryl, or a -CH₂-substituted silyl;
R⁶, R⁷, R⁸ and R⁹ are independently H, a substituted or unsubstituted aliphatic group, or a substituted or unsubstituted aromatic group;
R¹¹ is independently OCH₂CH₂CN, SCH₂CH₂CN, OCH₂CH=CH₂, or OCH₃;
R¹² is a substituted or unsubstituted aliphatic group, or a substituted or unsubstituted aromatic group;
X is independently H, an alkyl, an O-alkyl, an N-alkyl, or a halogen;
Y is independently H, NHR¹³, a halogen, CN, CF₃ or a hydroxyl group protected with an acyl protecting group, an ether protecting group or a silyl protecting group, or forms an X-Y bond with X;
R¹³ is independently H, an alkyl, a carbamate, an amide group, or a substituted silyl;
Z is independently O or S; and
n is an integer of 0 or more and 4 or less.

2. The optically active segment for use in synthesis of a stereocontrolled oligonucleotide according to claim 1, wherein in the case where B in formula (I) is a nucleoside protected with a protecting group, the protecting group is an acyl protecting group.

3. The optically active segment for use in synthesis of a stereocontrolled oligonucleotide according to claim 1,
wherein, in formula (I),
R¹ is an alkyloxy, methyl, trifluoromethyl, phenyl, or phenylacetyl group;
X is H;
Y is H or a hydroxyl group protected with a t-butyldimethylsilyl group;
Z is O; and
R³ is an isopropyl group.

4. A method for producing an optically active segment for use in synthesis of a stereocontrolled oligonucleotide, represented by the following formula (I): 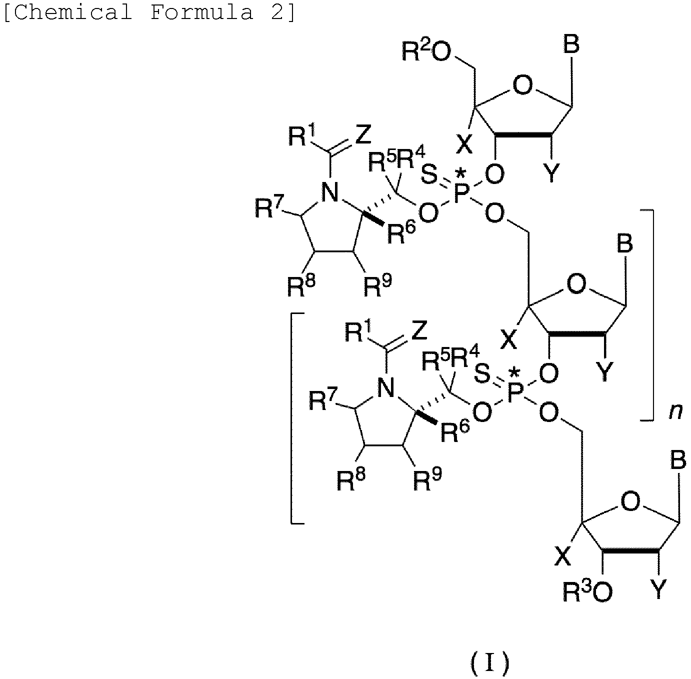
wherein B is independently a nucleoside base unprotected or protected with a protecting group;
R¹ is a substituted or unsubstituted aliphatic group, a substituted or unsubstituted aromatic group, or a substituted or unsubstituted heteroaryl group;
R³ is -P(R¹¹){N(R¹²)₂} in the case where R² is a protecting group removable under acidic conditions or a silyl protecting group, or R³ is a protecting group removable under acidic conditions or a silyl protecting group in the case where R² is -P(R¹¹){N(R¹²)₂};
R⁴ and R⁵ are independently H, an alkyl, an alkenyl, a substituted or unsubstituted aromatic group, a substituted or unsubstituted heteroaryl group, a -CH₂-substituted or unsubstituted aryl, or a -CH₂-substituted silyl;
R⁶, R⁷, R⁸ and R⁹ are independently H, a substituted or unsubstituted aliphatic group, or a substituted or unsubstituted aromatic group;
R¹¹ is independently OCH₂CH₂CN, SCH₂CH₂CN, OCH₂CH=CH₂, or OCH₃;
R¹² is a substituted or unsubstituted aliphatic group, or a substituted or unsubstituted aromatic group;
X is independently H, an alkyl, an O-alkyl, an N-alkyl, or a halogen;
Y is independently H, NHR¹³, a halogen, CN, CF₃ or a hydroxyl group protected with an acyl protecting group, an ether protecting group or a silyl protecting group, or forms an X-Y bond with X;
R¹³ is independently H, an alkyl, a carbamate, an amide group, or a substituted silyl;
Z is independently O or S; and
n is an integer of 0 or more and 4 or less;
the method comprising:
(a) a step of reacting a nucleoside represented by the following formula (II): 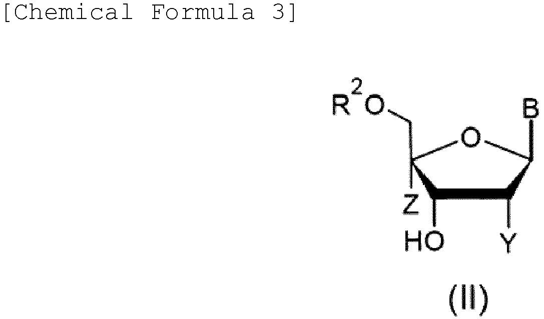 wherein R² is a protecting group removable under acidic conditions or a silyl protecting group,
with a compound represented by the following formula (III): 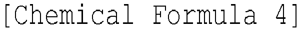 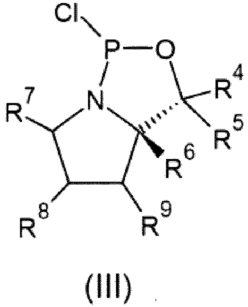 to prepare a compound having a structure represented by the following formula (IV): 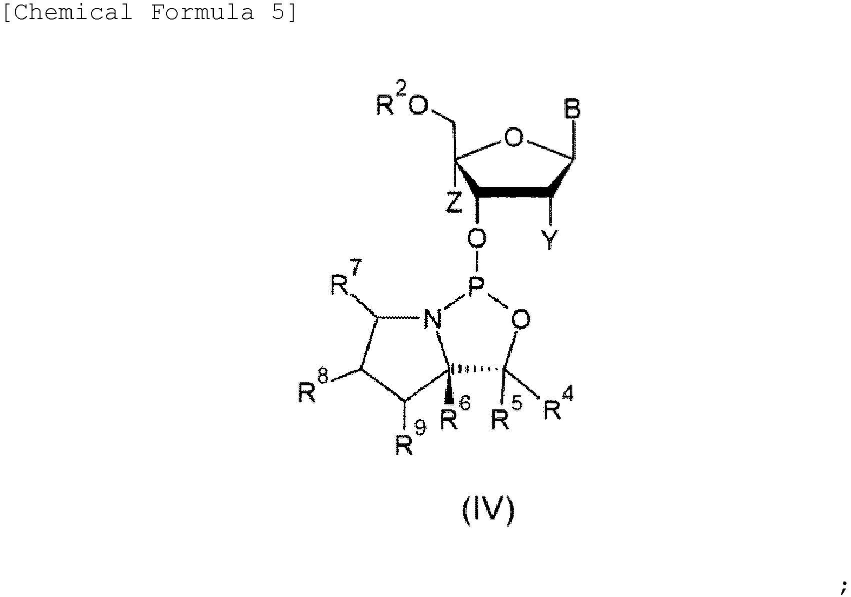
(b) a step of reacting the compound having a structure represented by formula (IV) with a compound having the structure of formula (V): 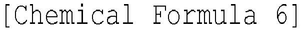 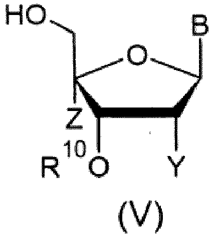 wherein R¹⁰ is an acyl, alkyloxycarbonyl, alkyl, acetal, or silyl protecting group,
and subsequently performing a sulfurization reaction to prepare a compound having a structure represented by the following formula (VI): 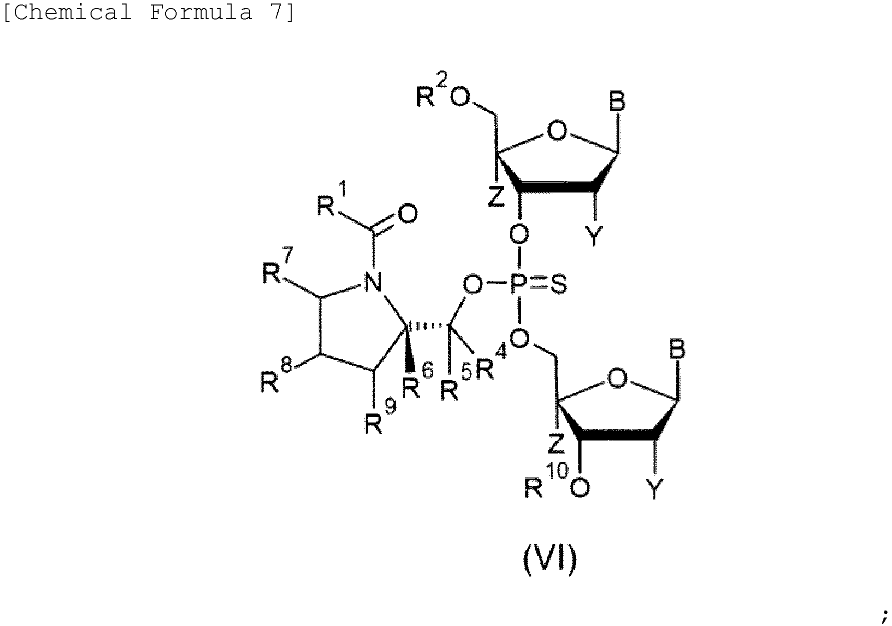
(c) a step of reacting a compound obtained through a deprotection reaction of 5'-hydroxyl group of the compound having the structure of formula (VI) with a compound having the structure of formula (IV) and then performing a sulfurization reaction 1 to 4 times, in the case of n=1 to 4 in formula (I); and
(d) a step of performing a deprotection reaction of the protecting group OR¹⁰ for 3'-hydroxyl group of the compound obtained in the step (b) or (c), and then reacting the product with a phosphitylating compound having a structure of R¹¹P{N(R¹²)₂}₂ to prepare a segment having the structure of formula (I).

5. The method according to claim 4, wherein in the case where B in formula (I) is a nucleoside protected with a protecting group, the protecting group is an acyl protecting group.

6. The method according to claim 4,
wherein, in formula (I), R¹ is an alkyloxy, methyl, trifluoromethyl, phenyl, or phenylacetyl group;
X is H;
Y is H or a hydroxyl group protected with a t-butyldimethylsilyl group; and
R³ is an isopropyl group.

7. A method for synthesizing a stereocontrolled oligonucleotide using the optically active segment for use in synthesis of a stereocontrolled oligonucleotide, represented by formula (I) according to claim 1, the method comprising:
(a) a condensation step of condensing an amidite moiety of the optically active segment represented by formula (I) with a hydroxyl group of a nucleoside or nucleotide, and
(b) a deprotection step of deprotecting the terminal protecting group of the segment for use in synthesis of an oligonucleotide condensed with a nucleoside or nucleotide in the condensation step.

8. The method according to claim 7, wherein each of the steps is performed in a solution.

9. The method according to claim 7, wherein each of the steps is performed on a solid-support.
